(19) **Europäisches Patentamt**

**European Patent Office**

**Office européen des brevets**

(11) **EP 0 885 855 B1**

(12) **EUROPÄISCHE PATENTSCHRIFT**

(45) Veröffentlichungstag und Bekanntmachung des Hinweises auf die Patenterteilung:
**26.03.2003 Patentblatt 2003/13**

(51) Int Cl.⁷: $C03C\ 10/16$, $A61K\ 6/033$, $A61K\ 6/06$

(21) Anmeldenummer: **98250186.8**

(22) Anmeldetag: **28.05.1998**

(54) **Transluzente Apatit-Glaskeramik**

Translucent apatite glass-ceramics

Vitrocéramiques d'apatite translucide

(84) Benannte Vertragsstaaten:
**AT CH DE FR GB IT LI SE**

(30) Priorität: **12.06.1997 DE 19725555**

(43) Veröffentlichungstag der Anmeldung:
**23.12.1998 Patentblatt 1998/52**

(73) Patentinhaber: **Ivoclar Vivadent AG**
**9494 Schaan (LI)**

(72) Erfinder:
• **Frank, Martin, Dipl.-Ing.**
**9494 Schaan (LI)**
• **Drescher, Helga**
**6800 Feldkirch (AT)**
• **Höland, Wolfram, Dr., Prof.**
**9494 Schaan (LI)**
• **Rheinberger, Volker, Dr.**
**9490 Vaduz (LI)**

(74) Vertreter: **UEXKÜLL & STOLBERG**
**Patentanwälte**
**Beselerstrasse 4**
**22607 Hamburg (DE)**

(56) Entgegenhaltungen:
**US-A- 4 536 480**          **US-A- 4 536 481**

**Beschreibung**

Die Erfindung betrifft eine transluzente Apatit-Glaskeramik, die sich insbesondere zum Einsatz in der restaurativen Zahnheilkunde und vor allem zur Beschichtung oder Verblendung von dentalen Restaurationen, wie Brücken oder Kronen, eignet.

Apatit-Glaskeramiken sind aus dem Stand der Technik bekannt. Sie werden üblicherweise als bioaktive Werkstoffe zum Knochenersatz in der Humanmedizin oder als Hauptkomponente von Glasionomerzementen in der Zahnmedizin verwendet.

Im Fall der bioaktiven Werkstoffe für den Knochenersatz haben sie jedoch sehr hohe CaO- und $P_2O_5$-Gehalte, um Bioaktivität, d.h. das direkte Verwachsen von Glaskeramik und lebendem Knochen, zu erreichen.

So ist aus der DE-A-40 20 893 ein glaskeramisches Implantationsmaterial bekannt, welches Apatit-Kristalle aufweist, aber auch sehr hohe Mengen an CaO enthält, um eine Bioaktivität zu erzielen.

Glaskeramiken für Glasionomerzemente besitzen ebenfalls hohe CaO-Gehalte und zumeist auch hohe Fluoridionen-Gehalte, um die gewünschte hohe Ionenabgabe im Mundmedium zu erzielen.

Diese beiden Arten von Apatit-Glaskeramiken sind jedoch weiß-opak und verfügen über eine hohe Ionenabgabe und/oder gleichzeitige Bioaktivität, so daß sie für die restaurative Zahnheilkunde nicht geeignet sind.

Eine Apatit-Glaskeramik für die restaurative Zahnheilkunde muß über optische Eigenschaften, wie Transluzenz und Färbung, verfügen, die denen des natürlichen Zahnes ähnlich sind. Ein lichtundurchlässiges, d.h. opakes Material ist hierfür nicht geeignet. Zudem ist Bioaktivität oder ein hohe Ionenabgabe nicht erwünscht, sondern vielmehr ist eine hohe chemische Stabilität gefordert, die sogar die des natürlichen Zahnes übersteigen soll.

In bekannten apatithaltigen Glaskeramiken für die restaurative Zahnheilkunde bildet regelmäßig nicht Apatit, sondern Leucit oder Mullit die Hauptkristallphase. Dies ist jedoch unerwünscht, da diese Kristalltypen es u.a. erschweren, die optischen Eigenschaften des vornehmlich aus nadelförmigem Apatit bestehenden natürlichen Zahnmaterials nachzuahmen.

Die EP-A-0 690 030 offenbart leucithaltige Phosphosilicat-Glaskeramiken, die in der Dentaltechnik eingesetzt werden können. Aufgrund des Leucit-Gehaltes haben sie allerdings sehr hohe lineare thermische Ausdehnungskoeffizienten, so daß sie für die Beschichtung von Werkstoffen mit niedrigen Ausdehnungskoeffizienten, wie z.B. Lithiumdisilicat-Glaskeramiken, nicht geeignet sind.

Ferner wird von A. Clifford und R. Hill (Journal of Non-Crystalline Solids 196 (1996) 346-351) eine Apatit-Glaskeramik beschrieben, die als weitere Kristallphase Mullit enthält. Der hohe Mullit-Gehalt bewirkt eine nur geringe Transluzenz.

Apatithaltige Glaskeramiken werden von S. Hobo et al. (Quintessence International 2 (1985) 135-141) und Wakasa et al. (J. Oral Rehabil. 17 (1990) 461-472 und J. Mat. Sci. Lett. 11 (1992) 339-340) für den restaurativen Zahnersatz offenbart. Diese Glaskeramiken verfügen jedoch über hohe CaO- und $P_2O_5$-Gehalte, so daß sie über nur geringe chemische Beständigkeit verfügen. Außerdem besitzen die Apatitkristalle in diesen Glaskeramiken keine nadelförmige Morphologie.

Weiter beschreibt die DE-A-34 35 348 apatithaltige Glaskeramiken zur Herstellung von Zahnkronen. Die Glaskeramiken enthalten jedoch keinerlei $Al_2O_3$ und sehr große Mengen an CaO, weshalb sie eine hohe Tendenz zum Ionenaustausch und demgemäß eine nur geringe chemische Stabilität zeigen. Überdies haben die Apatit-Kristalle nicht die nadelförmige Morphologie wie sie Apatit-Kristallen des natürlichen Zahnmaterials zu eigen ist.

Glaskeramiken mit guter chemischer Beständigkeit sind in EP-A-0 695 726 als Alkali-Zink-Silicat-Glaskeramiken offenbart. Diese Glaskeramiken besitzen jedoch den Nachteil, daß sie keinen Apatit, sondern als Kristallphase Leucit enthalten. Infolge des hohen Ausdehnungskoeffizienten von Leucit eignen sich die Glaskeramiken daher in der Regel nicht als Beschichtungen für Substrate mit niedrigem Ausdehnungskoeffizienten, wie insbesondere Lithiumdisilicat-Glaskeramiken. Die Glaskeramik enthält ferner zwingend ZnO, um ein gute chemische Stabilität zu erreichen.

Der Erfindung liegt demgemäß die Aufgabe zugrunde, eine Apatit-Glaskeramik zur Verfügung zu stellen, die in ihren optischen Eigenschaften und insbesondere ihrer hohen Transluzenz natürlichem Zahnmaterial ähnelt und dabei Apatit-Kristalle aufweist, die in ihrer Morphologie der der Carbonat-Apatit-Kristalle des natürlichen Zahnmaterials ähneln, jedoch eine höhere chemische Stabilität als diese aufweisen und damit der Glaskeramik eine ausgezeichnete chemische Stabilität verleihen. Weiterhin soll die Apatit-Glaskeramik einen geringen thermischen Ausdehnungskoeffizienten aufweisen und sich daher insbesondere als Dentalmaterial und vor allem als Beschichtung oder Verblendung von dentalen Restaurationen, wie Kronen oder Brücken, aus Lithiumdisilicat eignen.

Diese Aufgabe wird überraschenderweise durch die transluzente Apatit-Glaskeramik nach den Ansprüchen 1 bis 9 gelöst.

Gegenstand der Erfindung sind ebenfalls das Verfahren zur Herstellung der Apatit-Glaskeramik nach Anspruch 10, das Dentalmaterial nach den Ansprüchen 11 bis 13, die Verwendung nach den Ansprüchen 14 bis 17 sowie die geformten Dentalprodukte nach den Ansprüchen 18 bis 20.

Die erfindungsgemäße Apatit-Glaskeramik ist dadurch gekennzeichnet, daß sie die folgenden Komponenten

enthält:

| Komponente | Gew.-% |
|---|---|
| $SiO_2$ | 45,0 bis 70,0 |
| $Al_2O_3$ | 5,0 bis 22,0 |
| $P_2O_5$ | 0,5 bis 6,5 |
| $K_2O$ | 3,0 bis 8,5 |
| $Na_2O$ | 4,0 bis 13,0 |
| CaO | 1,5 bis 11,0 |
| F | 0,1 bis 2,5 |

und die Hauptkristallphase durch nadelförmige Apatit-Kristalle gebildet ist.

Die erfindungsgemäße Glaskeramik kann zusätzlich noch mindestens eine der folgenden Komponenten enthalten:

| Komponente | Gew. -% |
|---|---|
| $B_2O_3$ | 0 bis 8,0 |
| $La_2O_3$ | 0 bis 5,0 |
| $Li_2O$ | 0 bis 5,0 |
| BaO | 0 bis 5,0 |
| MgO | 0 bis 5,0 |
| ZnO | 0 bis 5,0 |
| SrO | 0 bis 7,0 |
| $TiO_2$ | 0 bis 4,0 |
| $ZrO_2$ | 0 bis 4,0 |
| $CeO_2$ | 0 bis 3,0. |

Die untere Grenze dieser zusätzlichen Komponenten ist üblicherweise 0,05 Gew.-%.

Für die einzelnen Komponenten der erfindungsgemäßen Apatit-Glaskeramik existieren bevorzugte Mengenbereiche. Diese können, sofern nicht anders angegeben, unabhängig voneinander gewählt werden und sind wie folgt:

| Komponente | Gew.-% |
|---|---|
| $SiO_2$ | 50,0 bis 68,0 |
| $Al_2O_3$ | 7,0 bis 21,0 |
| $P_2O_5$ | 0,5 bis 4,0 |
| $K_2O$ | 4,0 bis 8,0 |
| $Na_2O$ | 4,0 bis 11,0 |
| CaO | 2,0 bis 8,0 |
| F | 0,2 bis 2,0 |
| $B_2O_3$ | 0,2 bis 4,0 |
| $La_2O_3$ | 0 bis 3,0 |
| $Li_2O$ | 0 bis 3,0 |
| BaO | 0 bis 4,0 |
| MgO | 0 bis 4,0 |
| ZnO | 0 bis 4,0 |
| SrO | 0 bis 5,0 |
| $TiO_2 + ZrO_2$ | 0,2 bis 5,0 |
| $CeO_2$ | 0 bis 2,0 |

Besonders bevorzugte Mengenbereiche für die einzelnen Komponenten der erfindungsgemäßen Apatit-Glaskeramik sind wie folgt und diese können unabhängig voneinander gewählt werden:

| Komponente | Gew.-% |
|---|---|
| $SiO_2$ | 54,0 bis 65,0 |
| $Al_2O_3$ | 8,0 bis 21,0 |
| $P_2O_5$ | 0,5 bis 3,5 |
| $K_2O$ | 5,0 bis 8,0 |
| $Na_2O$ | 6,0 bis 11,0 |
| $CaO$ | 2,0 bis 6,0 |
| $F$ | 0,3 bis 1,5 |
| $B_2O_3$ | 0,2 bis 3,0 |
| $La_2O_3$ | 0 bis 2,0 |
| $Li_2O$ | 0 bis 2,0 |
| $BaO$ | 0 bis 3,0 |
| $MgO$ | 0 bis 3,0 |
| $ZnO$ | 0 bis 3,0 |
| $SrO$ | 0 bis 4,0 |
| $TiO_2$ | 0,5 bis 2,0 |
| $ZrO_2$ | 0,5 bis 3,0 |
| $CeO_2$ | 0,1 bis 1,5. |

Alle vorstehenden Mengen in Gew.-% beziehen sich auf die Glaskeramik.

Die erfindungsgemäße Glaskeramik kann weiter z.B. übliche Farbkomponenten zur Anpassung an die Farbe des natürlichen Zahnmaterials eines Patienten enthalten.

Durch Rasterelektronenmikroskop- und Röntgenbeugungsuntersuchungen konnte festgestellt werden, daß in der Glaskeramik Apatit, wie Hydroxy- und/oder Fluor-Apatit, die Hauptkristallphase bildet. Die Apatitkristalle sind erwiegend nadelförmig gewachsen. Die Apatitkristalle sind in ihrer größten Ausdehnung bevorzugt kleiner als 35 μm, insbesondere kleiner als 15 μm und besonders bevorzugt kleiner als 5 μm.

Durch die ausgeschiedenen Apatitkristalle, die in ihrem Aussehen den Carbonat-Apatit-Kristallen des natürlichen Zahnmaterials ähneln, werden die optischen Eigenschaften der Glaskeramik gesteuert. So ist es möglich, daß eine Glaskeramik mit einem Aussehen erzeugt wird, welches dem des Dentin oder Schmelzes des Zahnes entspricht. Gleichzeitig wird eine optische Tiefenwirkung in der Glaskeramik erzielt, wie es durch andere Kristall-Typen nicht möglich ist.

Bei der erfindungsgemäßen Glaskeramik sind röntgenographisch keine Leucitkristalle nachweisbar, allerdings können Nebenkristallphasen, wie z.B. Natrium-Calcium-Orthophosphat vom Typ $NaCaPO_4$ vorhanden sein.

Die erfindungsgemäße Glaskeramik zeichnet sich durch eine sehr hohe Transluzenz aus. Zur Quantifizierung der Transluzenz wurde nach dem in den Beispielen beschrieben Meßverfahren der CR-Wert bestimmt. Der CR-Wert, auch als Kontrastverhältnis bezeichnet, gibt das Verhältnis der Lichtreflektion eines Probekörpers der Glaskeramik auf schwarzem Hintergrund zu der Messung der Lichtreflektion des Probekörpers auf weißem Hintergrund an und dient somit als Maß für die Lichtdurchlässigkeit eines Materials. Der CR-Wert ist durch die folgende Formel definiert:

$$CR = Y_b / Y_w$$

mit

CR = Kontrastverhältnis,
$Y_b$ = Lichtreflektion des Probekörpers auf schwarzem Hintergrund, und
$Y_w$ = Lichtreflektion des Probekörpers auf weißem Hintergrund.

Der CR-Wert liegt stets im Bereich von 0 bis 1, wobei CR = 0 für eine Opazität von 0% und demnach ein vollständig transluzentes Material und CR = 1 für eine Opazität von 100% und demnach ein vollständig opakes, d.h. lichtundurchlässiges Material steht.

Die erfindungsgemäße Glaskeramik hat üblicherweise einen CR-Wert von 0 bis 0,9 und bevorzugt von 0,1 bis 0,75.

Ein weiterer besonderer Vorteil der erfindungsgemäßen Glaskeramik besteht darin, daß durch deren besondere Zusammensetzung in Kombination mit den speziellen ausgeschiedenen Apatit-Kristallen eine hohe chemische Stabilität erreicht wird, ohne daß zwingend ZnO eingebaut werden muß.

Es wird vermutet, daß diese Stabilität auf den sehr hohen Kristallinitätsgrad sowie die Bildung von Hydroxy- und Fluorapatit zurückzuführen ist. So ist die Stabilität der ausgeschiedenen Fluor- oder Hydroxy-Apatit-Kristalle höher als die des recht instabilen Carbonat-Apatits, der im natürlichen Zahnmaterial vorkommt.

Es hat sich gezeigt, daß die erfindungsgemäße Apatit-Glaskeramik den konventionellen apatithaltigen Glaskeramiken hinsichtlich der chemischen Beständigkeit überlegen ist. Eine besonders hohe chemische Stabilität kann überraschenderweise dann erzielt werden, wenn das Mol-Verhältnis von CaO zu $P_2O_5$ zu F in der Glaskeramik 1 zu (0,020 bis 1,5) zu (0,03 bis 4,2), insbesondere 1 zu (0,1 bis 0,5) zu (0,1 bis 1) beträgt.

Es ist weiterhin hervorzuheben, daß die Glaskeramik $B_2O_3$-frei erzeugt werden kann. Durch den Zusatz von $B_2O_3$ ergibt sich jedoch der Vorteil, daß das gesamte Sinterverhalten der Glaskeramik verbessert wird und eine Sinterung im bevorzugten Temperaturbereich von 650 °C bis 1050 °C ablaufen kann.

Die Apatit-Glaskeramik hat üblicherweise einen recht niedrigen thermischen Ausdehnungskoeffizienten von 6,0 bis 12,0 $\times$ $10^{-6}K^{-1}$, gemessen im Temperaturbereich von 100 °C bis 400 °C.

Zur Herstellung der erfindungsgemäßen Apatit-Glaskeramik wird

a) ein Ausgangsglas, welches die oben angegebenen Komponenten enthält, bei Temperaturen von 1200 °C bis 1650 °C erschmolzen,

b) die erhaltene Glasschmelze unter Bildung eines Glasgranulates in Wasser eingegossen,

c) das Glasgranulat gegebenenfalls zu einem Glaspulver mit einer mittleren Korngröße von 1 bis 450 μm, bezogen auf die Teilchenzahl, zerkleinert, und

d) das Glasgranulat oder das Glaspulver einer thermischen Behandlung von mehr als 900 °C und bis zu 1200 °C für eine Dauer von 30 Minuten bis 6 Stunden unterzogen.

In Stufe (a) wird zunächst ein Ausgangsglas erschmolzen, indem geeignete Ausgangsmaterialien, wie zum Beispiel Carbonate, Oxide und Fluoride, innig miteinander vermischt und auf die angegebenen Temperaturen erwärmt werden.

Dann wird in Stufe (b) die erhaltene Glasschmelze durch Eingießen in Wasser abgeschreckt und dadurch zu einem Glasgranulat umgewandelt. Diese Vorgehensweise wird üblicherweise auch als Fritten bezeichnet.

Gegebenenfalls wird das Glasgranulat anschließend in Stufe (c) zerkleinert und insbesondere mit üblichen Mühlen auf die gewünschte Korngröße gemahlen. Dabei hat das erhaltene Glaspulver bevorzugt eine mittlere Korngröße von 1 bis 450 μm, bezogen auf die Teilchenzahl.

In Stufe (d) wird das Glasgranulat oder gegebenenfalls das Glaspulver einer thermischen Behandlung bei einer Temperatur im Bereich von mehr als 900 °C bis 1200 °C für eine Dauer von 30 Minuten bis 6 Stunden, vorzugsweise 30 Minuten bis 3 Stunden, unterzogen. Eine Temperatur von mehr als 900 °C ist erforderlich, da es bei tieferen Temperaturen nicht zu der Ausbildung der Apatitkristalle in der gewünschten Form und Menge kommt.

Bei der Wärmebehandlung findet eine Volumenkristallisation statt. Diese führt zu einer homogenen Verteilung der Apatitkristalle innerhalb der gesamten Glaskeramik, im Gegensatz zur Leucitkristallisation, die nur an den inneren Oberflächen eines Glaspulvers stattfinden kann.

Der in Stufe (b) beschriebene Vorgang des Glasfrittens ist verantwortlich für das Einfrieren einer Glasstruktur mit sehr kleinen (< 100 nm) tropfenförmigen Ausscheidungen, die extrem dicht gepackt und fein verteilt vorliegen. Selbst im Rasterelektronenmikroskop ist bei 30000-facher Vergrößerung eine Restglasmatrix nicht mehr zu erkennen. Es wird angenommen, daß die bei der anschließenden thermischen Behandlung erfolgende Apatitkristallisation über diese Ausscheidungen verläuft, die daher als Primärkeime angesehen werden können.

Durch rasterelektronenmikroskopische und Röntgenbeugungsuntersuchungen konnte festgestellt werden, daß Apatit, vorzugsweise Fluor-Apatit, die Hauptkristallphase bildet. Die Größe der erhalten Kristalle kann dabei durch die gewählte Temperatur und die Dauer der thermischen Behandlung gesteuert werden. Zusätzlich zu den Apatitkristallen können je nach chemischer Zusammensetzung des eingesetzten Ausgangsglases weitere Kristallphasen gebildet werden. Neben den verschiedenen Kristallphasen können auch mikroheterogene Entmischungsbereiche, d.h. verschiedene Glasphasen vorliegen. Diese Bereiche sind im Rasterelektronenmikroskop als kleine mikroheterogene Tropfenglasphasen mit einer Größe von ca. 20 bis 400 nm erkennbar. Die auftretenden Tropfenglasphasen beeinflussen zusammen mit den Kristallen die optischen Eigenschaften der erfindungsgemäßen Glaskeramiken, wie z.B. Opaleszenz und Transluzenz.

Überraschenderweise können die optischen Eigenschaften der erfindungsgemäßen Apatit-Glaskeramik von glasig transparent bis weißlich trüb eingestellt werden. Dies ist für den Einsatz als Dentalmaterial oder Komponente davon zwingend erforderlich, um alle unterschiedlichen Erscheinungsformen des natürlichen Zahnes reproduzierbar herstellen zu können. Die feinen Apatitkristalle im Gefüge der erfindungsgemäßen Glaskeramik bewirken in Bezug auf Optik und strukturellen Aufbau eine sehr große Ähnlichkeit zum natürlichen Zahn.

Die erfindungsgemäße Apatit-Glaskeramik wird daher insbesondere als Dentalmaterial und bevorzugt als Komponente von Dentalmaterial eingesetzt.

Bei Einsatz der Apatit-Glaskeramik als Komponente von Dentalmaterial können durch geeignete Wahl ihrer Zusammensetzung sowie der Art der weiteren Komponenten Dentalmaterialien erhalten werden, bei denen wichtige Eigenschaften, wie z.B. Verarbeitungstemperatur, optische Eigenschaften, thermischer Ausdehnungskoeffizient und chemische Beständigkeit, genau an die jeweiligen Anforderungen angepaßt sind. Mit reiner Glaskeramik ist dies häufig nicht möglich.

Eine Kombination der gewünschten Eigenschaften kann bei der erfindungsgemäßen Apatit-Glaskeramik dadurch erzielt werden, daß sie mit Gläsern und/oder anderen Glaskeramiken gemischt wird. Dabei ist es bevorzugt, daß das Dentalmaterial 10 bis 90 Gew.-% der Apatit-Glaskeramik enthält.

Insbesondere ist es möglich, die erfindungsgemäße Glaskeramik gezielt als Mittel zum Verändern der optischen Eigenschaften von Gläsern oder anderen Glaskeramiken einzusetzen. Bei Dentalkeramik ist es das Bestreben, ein ausgewogenes Verhältnis zwischen Transluzenz und Helligkeit zu erreichen, das den Verhältnissen beim natürlichen Zahn möglichst nahe kommt. Ein Zahnersatz mit für das Auge hellem Aussehen sollte gleichzeitig eine hohe Transluzenz aufweisen.

Mit herkömmlichen Trübungsmitteln, wie $SnO_2$, läßt sich die gewünschte Helligkeit nur erreichen, wenn auf der Seite der Transluzenz Abstriche gemacht werden, d.h. eine dem natürlichen Zahn ähnliche Helligkeit führt zu einer Transluzenz, die geringer ist, als die des natürlichen Zahns.

Wenn man als Trübungsmittel hingegen eine erfindungsgemäße kristalline Apatit-Glaskeramik verwendet, kann man gleichzeitig die gewünschte Helligkeit und eine dem natürlichen Zahn ähnliche, hohe Transluzenz erhalten. In der kristallinen Apatit-Glaskeramik sind Kristalle von im allgemeinen bis zu 15 $\mu$m Größe, vorzugsweise bis zu 5 $\mu$m Korngröße, eingebettet.

Bevorzugt enthält das erfindungsgemäße Dentalmaterial zusätzlich zu der Apatit-Glaskeramik mindestens ein Glas und/oder eine Glaskeramik der Systeme Alkali-Silicat, Alkali-Erdalkali-Silicat, Alkali-Alumosilicat, Alkali-Zink-Borosilicat, Phosphosilicat oder Alumo-Fluor-Borosilicat. Bevorzugte Glaskeramiken und Gläser dieser Art sind im folgenden angegeben, wobei sich die Angaben in Gew.-% auf die jeweilige Glaskeramik oder das jeweilige Glas beziehen.

- Leucithaltige Phosphosilicat-Glaskeramik der Zusammensetzung:

  $SiO_2$ 49,0-57,5 Gew.-%, $Al_2O_3$ 11,4-21,0 Gew.-%, $P_2O_5$ 0,5-5,5 Gew.-%, CaO 2,5-11,5 Gew.-%, $K_2O$ 9,0-22,5 Gew.-%, $Na_2O$ 1,0-9,5 Gew.-%, $Li_2O$ 0-2,5 Gew.-%, $B_2O_3$ 0-2,0 Gew.-%, $TiO_2$ 0-3,0 Gew.-%, $ZrO_2$ 0,8-8,5 Gew.-%, $CeO_2$ 0-3,0 Gew.-%, F 0,25-2,5 Gew.-%, $La_2O_3$ 0-3,0 Gew.-%, ZnO 0-3,0 Gew.-%, BaO 0-3,0 Gew.-%, MgO 0-3,0 Gew.-% und SrO 0-3,0 Gew.-%.

- Opaleszierende Gläser der Zusammensetzung:

  $SiO_2$ 48,0-66,0 Gew.-%, $B_2O_3$ 0-1,0 Gew.-%, $Me(III)_2O_3$ 5,8-20,0 Gew.-%, $Me(I)_2O$ 6,0-22,0 Gew.-%, Me(II)O 3,5-16,0 Gew.-%, $Me(IV)O_2$ 0,5-10,0 Gew.%, $P_2O_5$ 0,5-5,0 Gew.-%, $CeO_2$ 0-3,0 Gew.-%, wobei die Menge an $Me(III)_2O_3$ durch 5,8-20,0 Gew.-% $Al_2O_3$ und 0-6,0 Gew.-% $La_2O_3$; die Menge an $Me(I)_2O$ durch 3,0-15,0 G-ew.-% $K_2O$, 3,0-12,0 Gew.-% $Na_2O$ und 0-2,5 Gew.-% $Li_2O$; die Menge an Me(II)O durch 0-10,0 Gew.-% CaO, 0-7,5 Gew.-% BaO, 0-9,0 Gew.-% MgO, 0-3,5 Gew.-% ZnO und 0-8,5 Gew.-% SrO; und die Menge an $Me(IV)O_2$ durch 0-5,0 Gew.-% $TiO_2$ und 0-5,0 Gew.-% $ZrO_2$ gebildet ist.

- Alkali-Zink-Silicat-Gläser der Zusammensetzung:

  $SiO_2$ 52,0-63,5 Gew.-%, $Me(III)_2O_3$ 8,5-13,0 Gew.%, $K_2O$ 0-20,5 Gew.-%, $Na_2O$ 1,5-20,0 Gew.-%, $Li_2O$ 0-5,0 Gew.%, ZnO 2,0-8,0 Gew.-%, Me(II)O 2,5-6,5 Gew.-%, $TiO_2$ + $ZrO_2$ 0,5-6,0 Gew.-%, $SnO_2$ 0-9,5 Gew.%, $P_2O_5$ 0-4,0 Gew.-%, F 0-2,0 Gew.-%, $CeO_2$ 0-3,0 Gew.-%, wobei die Menge an $Me(III)_2O_3$ durch 0-13 Gew.-% $Al_2O_3$ und 0-9,5 Gew.-% $La_2O_3$; und die Menge an Me(II)O durch 0-3,5 Gew.-% CaO, 0-4,5 Gew.-% BaO und 0-5,0 Gew.-% MgO gebildet ist.

Besonders bevorzugt wird jedoch mindestens ein nach üblichen Verfahren herstellbares Alkali-Silicat-Glas der folgenden Zusammensetzung 55,0-71,0 Gew.-% $SiO_2$, 5,0-16,0 Gew.-% $Al_2O_3$, 0,2-10,0 Gew.-% $B_2O_3$, 4,5-10,0 Gew.-% $K_2O$, 3,0-14,0 Gew.-% $Na_2O$, 0-4,0 Gew.-% $Li_2O$, 0-3,0 Gew.-% CaO, 0-5,0 Gew.-% BaO, 0-4,0 Gew.-% ZnO, 0,2-5,0 Gew.-% $ZrO_2$ + $TiO_2$, 0-2,0 Gew.-% $CeO_2$, 0-3,0 Gew.-% F und 0-0,6 Gew.-% $P_2O_5$ zusammen mit der Apatit-Glaskeramik verwendet. Dabei sind Gew.-%-Angaben auf das Glas bezogen. Mischungen von der Apatit-Glaskeramik mit mindestens einem Glas dieser Zusammensetzung ergeben Dentalmaterialien, die sich besonders gut als Beschichtungen für keramische Suprastrukturen und damit zur Herstellung von vollkeramischen Dentalprodukten mit zahnähnlichen optischen Eigenschaften und hoher chemischer Stabilität eignen.

Bevorzugt werden Gläser eingesetzt, die bei der weiteren Verarbeitung des Dentalmaterials zu Dentalprodukten und insbesondere bei einem Sintern oder sonstigem Erhitzen auf 600 °C bis 1000 °C für bis zu 2 h nicht kristallisieren. Vorteilhaft sind Gläser, die eine Sintertemperatur im Bereich von 650 °C bis 1050 °C aufweisen.

Das erfindungsgemäße Dentalmaterial wird bevorzugt zur Beschichtung eines Substrates, insbesondere einer dentalen Krone oder Brücke, eingesetzt. Dabei wird das Dentalmaterial insbesondere aufgesintert, um die gewünschte Beschichtung zu erzielen.

Bei Verwendung als Beschichtungs- oder Verblendmaterial wird die Apatit-Glaskeramik üblicherweise zuerst zu einem Pulver mit einer mittleren Korngröße von 5 bis 80 µm, bezogen auf die Teilchenzahl, zerkleinert. Dieses Pulver wird ggf. mit Zusätzen, wie Farbkomponenten und insbesondere Gläsern oder weiteren Glaskeramiken, sowie wäßrigen Lösungen zum Anmischen versetzt, und die erhaltene Mischung wird auf das Substrat aufgebracht und in gewünschter Weise geformt. Nach Formung erfolgt schließlich bei Temperaturen von 650 °C bis 1050 °C ein Sintern zu dem beschichteten, geformten Dentalprodukt.

Es besteht allerdings auch die Möglichkeit, eine aus der erfindungsgemäßen Glaskeramik gefertigte Dentalrestauration auf ein Substrat zu kleben.

Die erfindungsgemäße Apatit-Glaskeramik kann als Schicht- oder Verblendmaterial für glaskeramische, vollkeramische, metallische oder auf einem Compositwerkstoff aufgebaute Dentalsuprastrukturen mit einem thermischen Ausdehungskoeffizienten von 7,0 bis 12,0 insbesondere von 8,0 bis 11,0 $\times$ $10^{-6}K^{-1}$ verwendet werden. Bevorzugt wird sie zur Beschichtung oder Verblendung von $ZrO_2$-Keramiken, $Al_2O_3$-Keramiken, $ZrO_2$/$Al_2O_3$-Keramiken, keramischen oder glaskeramischen Compositwerkstoffen und Titan verwendet.

Besonders vorteilhaft wird sie jedoch zur Verblendung von Suprastrukturen auf Basis von Lithiumdisilicat-Glaskeramik eingesetzt, um auf diese Weise ästhetisch sehr ansprechende vollkeramische Dentalprodukte herzustellen, die nicht nur eine ausgezeichnete chemische Stabilität haben, sondern sich auch durch eine sehr hohe Festigkeit auszeichnen.

Als besonders geeignet haben sich dabei Lithiumdisilicat-Glaskeramiken der folgenden Zusammensetzung erwiesen, die durch Erschmelzen entspechender Ausganggläser, Fritten und Wärmebehandlung bei 400 °C bis 1100 °C erhalten werden:

| Komponente | Gew.-% |
|---|---|
| $SiO_2$ | 57,0 bis 80,0 |
| $Al_2O_3$ | 0 bis 5,0 |
| $La_2O_3$ | 0,1 bis 6,0 |
| MgO | 0 bis 5,0 |
| ZnO | 0 bis 8,0 |
| $K_2O$ | 0 bis 13,5 |
| $Li_2O$ | 11,0 bis 19,0 |
| $P_2O_5$ | 0 bis 11,0 |

wobei

(a) $Al_2O_3$ + $La_2O_3$      0,1 bis 7,0 Gew.-% und
(b) MgO + ZnO      0,1 bis 9,0 Gew.-%

ausmachen.

Für die Herstellung von Beschichtungen ist erfindungsgemäßes Dentalmaterial vorteilhaft, daß einen linearen thermischen Ausdehnungskoeffizienten hat, der kleiner ist als der des zu beschichtenden Substrates. Besonders vorteilhaft sind Dentalmaterialien, deren Ausdehungskoeffizient nicht mehr als 3,0 $\times$ $10^{-6}K^{-1}$ kleiner ist als der des Substrates. Bevorzugt hat das Dentalmaterial einen linearen thermischen Ausdehnungskoeffizienten von 5,5 bis 12,5 x $10^{-6}K^{-1}$, gemessen im Temperaturbereich von 100 °C bis 400 °C.

Die Apatit-Glaskeramik und das erfindungsgemäße Dentalmaterial können zusammen mit den ggf. vorhandenen Zusätzen zu geformten Dentalprodukten in üblicher Weise verarbeitet werden. Als erfindungsgemäße geformte Dentalprodukte, die einen Gehalt an der erfindungsgemäßen Apatit-Glaskeramik oder dem erfindungsgemäßen Dentalmaterial aufweisen, kommen neben gewünscht geformten Rohlingen insbesondere Dentalrestaurationen, wie z.B. ein Inlay, ein Onlay, eine Brücke, ein Stiftaufbau, eine Verblendung, eine Schale, ein Veneer, eine Facette, eine Füllung, ein Verbinder, eine Krone oder eine Teilkrone in Frage.

Im Gegensatz zu konventionellen Glaskeramiken konnte in der erfindungsgemäßen Apatit-Glaskeramik eine Leucit-Kristallphase nicht nachgewiesen werden. Diese ist auch unerwünscht, da sie infolge des hohen Ausdehnungskoeffizienten von Leucit einer Glaskeramik ebenfalls einen hohen thermischen Ausdehungskoeffizienten von üblicherweise mehr als $12,5 \times 10^{-6}K^{-1}$ verleihen würde. Bei Einsatz von leucithaltiger Glaskeramik zur Beschichtung eines Substrat, das einen Ausdehnungskoeffizienten von weniger als $12,5 \times 10^{-6}K^{-1}$ besitzt, wie z.B. $ZrO_2$ oder Lithiumdisilicat-Glaskeramik, kommt es daher auch zu sehr hohe Spannungen, die sich in Rissen und Abplatzungen äußern. Diese Nachteile zeigt die erfindungsgemäße Glaskeramik infolge ihres niedrigen Ausdehnungskoeffizienten nicht, so daß sie sich sehr gut zur Beschichtung von Substraten mit niedrigen Ausdehnungskoeffizienten eignet.

Die Erfindung wird nachstehend anhand von Beispielen näher erläutert.

## Beispiele

### Beispiele 1 bis 17

Es wurden insgesamt 17 verschiedene erfindungsgemäße Glaskeramiken hergestellt. Sie hatten die in der Tabelle I angegebenen chemischen Zusammensetzungen und Molverhältnisse von CaO zu $P_2O_5$ zu F und besaßen alle chemische Stabilitäten von weniger als 100 µg/cm$^2$ Masseverlust gemäß ISO 6872:1995.

EP 0 885 855 B1

Tabelle_I: Zusammensetzung erfindungsgemäßer Glaskeramiken (Mengen in Gew.-%) und jeweiliges Molverhältnis CaO:$P_2O_5$:F

| Beispiel Nr. | $SiO_2$ | $Al_2O_3$ | $P_2O_5$ | CaO | F | $K_2O$ | $Na_2O$ | $Li_2O$ | $B_2O_3$ | $TiO_2$ | $ZrO_2$ | $CeO_2$ | BaO | ZnO | SrO | Molverhältnis CaO : $P_2O_5$ : F |
|---|---|---|---|---|---|---|---|---|---|---|---|---|---|---|---|---|
| 1 | 64,5 | 8,4 | 1,1 | 2,8 | 0,7 | 6,6 | 9,6 | --- | 2,2 | 1,2 | 0,4 | --- | --- | 2,5 | --- | 1 : 0,156 : 0,719 |
| 2 | 56,9 | 19,7 | 2,6 | 1,7 | 2,5 | 8,0 | 8,6 | --- | --- | --- | --- | --- | --- | --- | --- | 1 : 0,6 : 4,2 |
| 3 | 45,2 | 21,9 | 3,2 | 4,5 | 0,6 | 7,5 | 13,0 | 0,4 | 2,0 | 0,5 | 0,7 | 0,5 | --- | --- | --- | 1 : 0,282 : 0,4 |
| 4 | 54,7 | 19,5 | 1,2 | 2,6 | 0,6 | 6,2 | 10,0 | --- | 2,0 | 1,0 | 1,7 | 0,5 | --- | --- | --- | 1 : 0,172 : 0,641 |
| 5 | 62,0 | 9,0 | 2,6 | 4,3 | 0,6 | 7,5 | 8,6 | --- | --- | 0,4 | 1,1 | 0,8 | --- | --- | 3,1 | 1 : 0,24 : 0,42 |
| 6 | 64,4 | 6,3 | 1,2 | 2,8 | 0,6 | 6,5 | 9,8 | --- | --- | 1,6 | 0,8 | 0,5 | 2,4 | 3,1 | --- | 1 : 0,172 : 0,656 |
| 7 | 69,7 | 5,1 | 3,7 | 5,3 | 0,8 | 3,2 | 4,1 | 5,0 | 0,9 | 0,8 | 0,8 | 0,6 | --- | --- | --- | 1 : 0,277 : 0,429 |
| 8 | 62,8 | 13,1 | 1,2 | 2,7 | 0,6 | 6,3 | 5,9 | --- | --- | --- | 1,7 | 0,5 | 1,8 | 3,4 | --- | 1 : 0,172 : 0,641 |
| 9 | 55,5 | 19,2 | 1,2 | 2,7 | 0,6 | 6,7 | 9,7 | --- | 0,3 | 1,4 | 2,2 | 0,5 | --- | --- | --- | 1 : 0,167 : 0,621 |
| 10 | 53,7 | 13,7 | 2,6 | 11,2 | 0,12 | 8,3 | 7,9 | 0,58 | --- | 0,7 | 0,7 | 0,5 | --- | --- | --- | 1 : 0,086 : 0,032 |
| 11 | 55,5 | 9,8 | 2,8 | 4,9 | 1,5 | 5,7 | 3,8 | 0,2 | 8,0 | 1,3 | 1,1 | 0,5 | 2,8 | 2,1 | --- | 1 : 0,224 : 0,897 |
| 12 | 55,4 | 19,9 | 0,5 | 2,0 | 0,4 | 7,4 | 7,3 | --- | --- | --- | 1,6 | 0,7 | 4,8 | --- | --- | 1 : 0,096 : 0,6 |
| 13 | 57,5 | 8,0 | 3,1 | 5,1 | 1,7 | 6,6 | 9,2 | --- | 3,3 | 1,3 | 0,8 | 0,8 | --- | 2,6 | --- | 1 : 0,241 : 1 |
| 14 | 59,2 | 7,9 | 3,0 | 5,1 | 0,6 | 6,8 | 9,6 | 0,3 | 1,0 | 1,5 | 2,5 | 0,5 | --- | 2,0 | --- | 1 : 0,233 : 0,35 |
| 15 | 54,4 | 19,5 | 2,6 | 1,7 | 2,5 | 7,9 | 8,6 | 0,4 | 0,4 | 0,7 | 0,8 | 0,5 | --- | --- | --- | 1 : 0,6 : 4,2 |
| 16 | 54,3 | 18,4 | 6,4 | 1,7 | 2,4 | 7,8 | 7,5 | 0,4 | 0,4 | 0,7 | --- | --- | --- | --- | --- | 1 : 1,485 : 4,16 |
| 17 | 50,1 | 18,2 | 0,6 | 10,9 | 0,15 | 7,7 | 8,5 | 0,7 | --- | 0,7 | 0,7 | 0,55 | --- | 1,2 | --- | 1 : 0,021 . 0,04 |

Zu ihrer Herstellung wurde jeweils ein entsprechendes Gemenge von geeigneten Oxiden, Carbonaten und Fluoriden in einem Platin/Rhodium-Tiegel bei einer Temperatur von 1550 °C bis 1600 °C während einer Homogenisierungszeit von 1 bis 1,5 Stunden erschmolzen. Die Glasschmelze wurde in Wasser abgeschreckt, und das gebildete Granulat des Ausgangsglases wurde getrocknet und auf eine mittlere Korngröße von weniger als 90 μm aufgemahlen.

Anschließend wurde das erhaltene Pulver des Ausgangsglases 30 Minuten bis 6 Stunden einer Wärmebehandlung bei mehr als 900 °C und bis zu 1200 °C unterworfen, woraufhin sich die Glaskeramik bildete.

Für einige der Glaskeramiken sind in Tabelle II ausgewählte Eigenschaften angegeben, die an Probekörpern aus der jeweiligen Glaskeramik bestimmt worden sind. Weiter finden sich in Tabelle II unter "Thermischer Behandlung" Angaben zu der konkret gewählten Wärmebehandlung des Ausgangsglases.

Die Beispiele verdeutlichen wie durch Veränderung der chemischen Zusammensetzung Glaskeramiken mit unterschiedlichen Eigenschaften erhalten werden können.

Tabelle II

| Beispiele | Thermische Behandlung [°C/h] | Brenntemperatur∗ [°C] | Tg [°C] | $\alpha$-Wert $\times 10^{-6} K^{-1}$ (100 °C -400 °C) | Optik | Säurebeständigkeit [μg/cm$^2$] |
|---|---|---|---|---|---|---|
| 1 | 1050 / 1 | 860 | 545 | 8,4 | milchig, leicht opal transluzent | 21 |
| 8 | 1000 / 1 | 1080 | 650 | 7,9 | sehr transluzent | 23 |
| 9 | 1020 / 1 | 1050 | 645 | 9,7 | sehr transluzent | 28 |
| 11 | 1000 / 1 | 890 | 547 | 6,6 | gelblich milchig, transluzent | 58 |
| 14 | 1050 / 1 | 870 | 541 | 9,4 | weißlich trüb, transluzent | 55 |

∗ Brenntemperatur = Temperatur, die bei der Prüfkörperherstellung durch Sinterung auf Quarz benutzt wurde (1 Minute Haltezeit, Vakuum)

Bestimmung des Ausdehungskoeffizienten $\alpha$

Zur Messung des linearen thermischen Ausdehnungskoeffizienten $\alpha$ wurde aus Pulver der jeweiligen Glaskeramik ein stäbchenförmiger Grünkörper hergestellt, der in einem Vakuumbrennofen mit einer Aufheizrate von 60 °C/min und einer Haltezeit von 1 Minute bei der jeweils angegebenen Brenntemperatur gesintert wurde. Anschließend wurde ein Glanzbrand ohne Vakuum bei einer um 20 °C höheren Endtemperatur und einer Haltezeit von 1 Minute durchgeführt. An dem erhaltenen Probekörper wurde der lineare thermische Ausdehnungskoeffizient bestimmt.

Bestimmung der Säurebeständigkeit

Die Säurebeständigkeit ist ein Maß für die chemische Stabilität gerade von im Dentalbereich eingesetzten Glaskeramiken, da diese in der Mundhöhle permanent der Einwirkung von sauren Substanzen ausgesetzt sind.

Die Säurebeständigkeit wurde gemäß der ISO-Vorschrift 6872:1995 bestimmt. Dazu wurden zunächst Probeplättchen mit einem Durchmesser von 12 mm und einer Dicke von 1 mm durch Zusammensintern von Glaskeramik-Granulat mit einer mittleren Teilchengröße von 90 μm hergestellt. Das Granulat wurde 1 Minute lang auf der Sintertemperatur gehalten. Dann wurden die Probeplättchen 16 Stunden lang in einer Soxhlet-Apparatur mit 4 Vol.-%iger wäßriger Essigsäure behandelt, und es wurde schließlich der eingetretene Masseverlust als Maß für die Säurebeständigkeit bestimmt.

In den folgenden Beispielen wurden Mischungen der Apatit-Glaskeramiken mit Zusatzkomponenten untersucht. Als Zusatzkomponenten einsetzbare Gläser und/oder andere Glaskeramiken hatten die aus Tabelle III ersichtliche Zusammensetzung.

Tabelle III: Zusammensetzung von Gläsern und Glaskeramiken als Zusatzkomponenten (Angaben in Gew.-%)

| Zusatzkomponente | $SiO_2$ | $Al_2O_3$ | $P_2O_5$ | CaO | F | $K_2O$ | $Na_2O$ | $Li_2O$ | $B_2O_3$ | $TiO_2$ | $ZrO_2$ | $CeO_2$ | BaO | ZnO |
|---|---|---|---|---|---|---|---|---|---|---|---|---|---|---|
| Alkali-Silicat-Glas (A) | 61,5 | 8,7 | --- | 1,0 | 1,7 | 7,0 | 8,8 | --- | 2,4 | 1,5 | 1,0 | 0,5 | 2,9 | 3,0 |
| Alkali-Silicat-Glas (B) | 61,4 | 8,5 | --- | 1,1 | 1,7 | 7,8 | 8,7 | 0,6 | 1,9 | 1,5 | 1,0 | 0,5 | 2,1 | 3,2 |
| Alkali-Silicat-Glas (C) | 62,3 | 8,7 | --- | 1,3 | 1,6 | 7,0 | 7,0 | 2,0 | 1,1 | 1,4 | 1,0 | 0,6 | 3,0 | 3,0 |
| Alkali-Silicat-Glas (D) | 70,8 | 8,6 | --- | 2,1 | 0,9 | 6,9 | 8,3 | 1,5 | 0,2 | 0,7 | --- | --- | --- | --- |
| Alkali-Silicat-Glas (E) | 63,4 | 6,2 | 0,4 | 1,7 | --- | 6,4 | 9,6 | --- | 3,7 | 1,7 | 1,1 | 0,5 | 2,3 | 3,0 |
| Alkali-Silicat-Glas (F) | 61,9 | 9,9 | --- | 1,1 | 1,5 | 5,8 | 3,7 | 0,2 | 8,0 | 1,4 | 1,1 | 0,5 | 2,8 | 2,1 |
| | | | | | | | | | | | | | | |
| Leucit-Phosphosilicat-Glaskeramik (G) | 56,8 | 13,6 | 2,6 | 3,7 | 0,3 | 10,8 | 7,5 | 0,2 | 0,3 | 0,4 | 1,1 | 1,0 | 0,9 | 0,8 |
| Alkali-Zink-Silicat-Glas (H) | 57,1 | 9,5 | --- | 1,9 | 0,9 | 9,6 | 9,3 | 1,7 | --- | --- | 1,0 | 1,0 | 3,9 | 4,1 |
| Opaleszierendes Glas (I) | 55,8 | 15,2 | 2,6 | 2,6 | --- | 11,0 | 9,6 | --- | 0,3 | --- | 1,9 | 1,0 | --- | --- |

EP 0 885 855 B1

### Beispiel 18

Dieses Beispiel beschreibt den Einsatz der erfindungsgemäßen Glaskeramik nach Beispiel 9 als Beschichtungsmaterial für keramische Suprastrukturen und somit zur Herstellung von vollkeramischen Dentalprodukten.

Zur Herstellung der Glaskeramik wurde Glaspulver entsprechender Zusammensetzung 1 Stunde lang bei 1020 °C wärmebehandelt. Die gebildete Glaskeramik wurde mittels Rasterelektronenmikroskopie untersucht, und die gebildeten Kristalle konnten mittels Röntgendiffraktometrie als nadelförmige Apatit-Kristalle identifiziert werden.

Zur geeigneten Einstellung des Ausdehnungskoeffizienten und der Sintertemperatur wurde die Glaskeramik mit den Alkali-Silicat-Gläsern (A) und (B) (siehe Tabelle III) gemischt.

Die Herstellung dieser Alkali-Silicat-Gläser erfolgte analog zu der oben in den Beispielen 1 bis 17 beschriebenen Herstellung der Ausgangsgläser.

Die Glaskeramik und die zwei Alkali-Silicat-Gläser wurden in Form von Pulvern einer mittleren Korngröße von weniger als 90 μm und in einem Gew.-Verhältnis von 40 % Apatit-Glaskeramik gemäß Beispiel 9 (siehe Tabelle II), 30 % Alkali-Silicat-Glas (A) und 30% Alkali-Silicat-Glas (B) gemischt.

Diese Mischung wurde zu einem stäbchenförmigen Grünkörper in einem Vakuumofen bei einer Aufheizgeschwindigkeit von 60 °C/min und einer Haltezeit von 1 min bei 870 °C gesintert. Für die so erhaltene Probe wurde ein thermischer Ausdehnungskoeffizient von 9,5 x $10^{-6}$K$^{-1}$, gemessen im Temperaturbereich von 100 °C bis 400 °C, bestimmt.

Damit konnte diese Mischung zum Aufsintern auf ein Substrat mit einem thermischen Ausdehnungskoeffizienten von 10,6 x $10^{-6}$K$^{-1}$, wie eine Lithiumdisilicat-Glaskeramik, bei einer vorteilhaften Verarbeitungstemperatur von 830 °C verwendet werden. Diese Verarbeitung auf dem zahntechnischen Substrat kann in der Regel bei 50 °C bis 100 °C tieferen Temperaturen als das Sintern auf Quarz erfolgen.

Die erhaltenen vollkeramischen Produkte zeichnen sich durch hohe chemische Stabilität, ästhetisches Aussehen sowie hohe Festigkeit aus.

### Beispiel 19

Analog zu Beispiel 18 können zur Erzielung von gewünschten Ausdehnungskoeffizienten und Sintertemperaturen auch unterschiedliche erfindungsgemäße Apatit-Glaskeramiken miteinander oder mit anderen Gläsern gemischt werden.

So wurde eine Pulver-Mischung aus 25 Gew.-% Glaskeramik gemäß Beispiel 4 (Wärmebehandlung bei 1020 °C), 50 Gew.-% Glaskeramik gemäß Beispiel 14 (Wärmebehandlung bei 1050 °C) und 25 Gew.-% des Alkali-Silicat-Glases (B) (siehe Tabelle III) hergestellt. Diese Mischung hatte eine vorteilhafte Sintertemperatur von lediglich 830 °C und einen Ausdehnungskoeffizienten von 9,5 x $10^{-6}$K$^{-1}$.

Die Mischung besaß hervorragende optische Eigenschaften und eignete sich ausgezeichnet als Aufbrennkeramik für ein vollkeramisches Dentalgerüst mit niedrigem Ausdehnungskoeffizienten.

### Beispiele 20 bis 27

In diesen Beispielen wurden weitere Mischungen von erfindungsgemäßen Apatit-Glaskeramiken mit Gläsern und Glaskeramiken untersucht.

Die Zusammensetzungen der einzelnen Mischungen sowie die durchgeführte thermische Behandlung zur Herstellung der jeweils eingesetzten Apatit-Glaskeramik sind in Tabelle IV aufgeführt.

Die für diese Mischungen bestimmten Eigenschaften sind ebenfalls in Tabelle IV wiedergegeben, und sie zeigen, daß durch geeignete Wahl der Komponenten Dentalmaterialien mit an den jeweiligen Anwendungszweck angepaßten Eigenschaften erhalten werden können.

EP 0 885 855 B1

Tabelle IV: Zusammensetzungen und Eigenschaften von Mischungen erfindungsgemäßer Apatit-Glaskeramiken mit Gläsern und/oder Glaskeramiken

| Beispiele | Zusammensetzung | Thermische Behandlung [°C/h] | Mischungs-verhältnis [in Gew.-%] | Brenn-temperatur [°C] | Tg [°C] | α-Wert × $10^{-6}K^{-1}$ (100 °C -400 °C) | Optik | Säurebe-ständigkeit [$\mu g/cm^2$] |
|---|---|---|---|---|---|---|---|---|
| 20 | Apatit-Glaskeramik 9 Alkali-Silicat-Glas (A) Alkali-Silicat-Glas (B) | 1000 / 1 --- --- | 30 35 35 | 880 | 528 | 9,5 | sehr transluzent | 34 |
| 21 | Apatit-Glaskeramik 14 Alkali-Silicat-Glas (A) | 1050 / 1 --- | 50 50 | 850 | 530 | 9,3 | milchig trüb, transluzent | 38 |
| 22 | Apatit-Glaskeramik 14 Alkali-Silicat-Glas (F) | 1020 / 1 --- | 50 50 | 870 | 542 | 8,0 | milchig, transluzent | < 100 |
| 23 | Apatit-Glaskeramik 8 Alkali-Silicat-Glas (C) | 1000 / 1 --- | 40 60 | 910 | 552 | 8,8 | sehr transluzent | 29 |
| 24 | Apatit-Glaskeramik 1 Alkali-Silicat-Glas (D) | 1050 / 1 --- | 70 30 | 850 | 539 | 8,7 | leicht milchig, leicht opal, transluzent | 26 |
| 25 | Apatit-Glaskeramik 9 Alkali-Zink-Silicat-Glas (H) | 1020 / 1 --- | 20 80 | 780 | 463 | 10,9 | transparent | 24 |
| 26 | Apatit-Glaskeramik 9 Opaleszierendes Glas (I) | 1020 / 1 --- | 50 50 | 1020 | 600 | 10,2 | sehr transluzent, leicht bräunlich opal | 27 |
| 27 | Apatit-Glaskeramik 14 Leucit-Phosphosilicat-Glaskeramik (G) | 1050 / 1 | 30 70 | 910 | 560 | 9,7 | weißlich, transluzent | 45 |

13

**Beispiel 28**

In diesem Beispiel wurde an ausgewählten erfindungsgemäßen Dentalmaterialien die Transluzenz quantitativ durch Bestimmung des CR-Wertes ermittelt.

Es wurde hierzu das Meßverfahren der British Standards Institution benutzt, weiches in der Prüfnorm für Dentalkeramik "BS 5612:1978" beschrieben ist.

Es wurden pro Material 5 Prüfkörper mit einem Durchmesser von 20 mm und einer Probendicke von 1,75 mm bei entsprechender Sintertemperatur gebrannt. Die Probekörper wurden mit nassem SiC-Pulver, Körnung 320, beschliffen, um die gewünschte Oberflächengüte zu erhalten (Oberflächenrauhigkeit Ra = 0,8 µm - 1,6 µm). Dabei ist es wichtig, daß die Planparallelität der gegenüberliegenden Seiten eine Toleranz von ± 0,01 mm nicht überschreitet, da das Meßergebnis in hohem Maße von der Schichtstärke abhängt. Die endgültige Probenhöhe/-dicke sollte $1,00 \pm 0,025$ mm betragen.

Die Probekörper wurden in einem Farbmeßinstrument Minolta-CR 300 in die vorgesehene Meßöffnung gelegt, und die Remission von jedem der 5 Probekörper wurde mit einer Blende von 10 mm gemessen. Bei der Messung dürfen die Proben nicht in optischem Kontakt mit dem Hintergrund sein, was ggf. durch Aufgabe eines Tropfens Glycerin auf den Hintergrund verhindert werden kann.

(a) Zur Ermittlung der Probenemission auf schwarzem Hintergrund $Y_b$ ($Y_{black}$) wurde ein schwarzes Plättchen mit nicht mehr als 4 % Reflexion benutzt.

(b) Zur Ermittlung der Probenemission auf weißem Hintergrund $Y_w$ ($Y_{white}$) wurde weißes Plättchen mit einer Reflexion von 80 % bis 85 % benutzt.

Aus den ermittelten Werten $Y_b$ und $Y_w$ wurde dann der Kontrastwert CR nach CR = $Y_b$ / $Y_w$ berechnet, und er war für die zwei untersuchten Materialien wie folgt:

Material 1: $CR_1$ = 0,13 -> 13 % Opazität
Material 2: $CR_2$ = 0,50 -> 50 % Opazität

Die Materialien hatten nachstehende Zusammensetzung:

Material 1: Zusammensetzung wie Mischung gemäß Beispiel 20

Material 2: 50 Gew.-% Mischung gemäß Beispiel 20 50 Gew.-% Apatit-Glaskeramik gemäß Beispiel 14 (Wärmebehandlung 1050 °C, 1 Stunde)

Die obigen Ergebnisse zeigen, daß durch geeignete Wahl der Zusammensetzung der Materialien die Transluzenz eingestellt werden kann.

**Patentansprüche**

**1.** Transluzente Apatit-Glaskeramik, **dadurch gekennzeichnet, daß** sie die folgenden Komponenten enthält:

| Komponente | Gew.-% |
|---|---|
| $SiO_2$ | 45,0 bis 70,0 |
| $Al_2O_3$ | 5,0 bis 22,0 |
| $P_2O_5$ | 0,5 bis 6,5 |
| $K_2O$ | 3,0 bis 8,5 |
| $Na_2O$ | 4,0 bis 13,0 |
| CaO | 1,5 bis 11,0 |
| F | 0,1 bis 2,5 |

und die Hauptkristallphase durch nadelförmige Apatit-Kristalle gebildet ist.

**2.** Apatit-Glaskeramik nach Anspruch 1, **dadurch gekennzeichnet, daß** sie mindestens eine der folgenden Kompo-

nenten enthält:

| Komponente | Gew.-% |
|---|---|
| $B_2O_3$ | 0 bis 8,0 |
| $La_2O_3$ | 0 bis 5,0 |
| $Li_2O$ | 0 bis 5,0 |
| BaO | 0 bis 5,0 |
| MgO | 0 bis 5,0 |
| ZnO | 0 bis 5,0 |
| SrO | 0 bis 7,0 |
| $TiO_2$ | 0 bis 4,0 |
| $ZrO_2$ | 0 bis 4,0 |
| $CeO_2$ | 0 bis 3,0. |

3. Apatit-Glaskeramik nach Anspruch 1 oder 2, **dadurch gekennzeichnet, daß** die Mengen der Komponenten, sofern nicht anders angegeben, unabhängig voneinander wie folgt sind:

| Komponente | Gew.-% |
|---|---|
| $SiO_2$ | 50,0 bis 68,0 |
| $Al_2O_3$ | 7,0 bis 21,0 |
| $P_2O_5$ | 0,5 bis 4,0 |
| $K_2O$ | 4,0 bis 8,0 |
| $Na_2O$ | 4,0 bis 11,0 |
| CaO | 2,0 bis 8,0 |
| F | 0,2 bis 2,0 |
| $B_2O_3$ | 0,2 bis 4,0 |
| $La_2O_3$ | 0 bis 3,0 |
| $Li_2O$ | 0 bis 3,0 |
| BaO | 0 bis 4,0 |
| MgO | 0 bis 4,0 |
| ZnO | 0 bis 4,0 |
| SrO | 0 bis 5,0 |
| $TiO_2 + ZrO_2$ | 0,2 bis 5,0 |
| $CeO_2$ | 0 bis 2,0 |

4. Apatit-Glaskeramik nach einem der Ansprüche 1 bis 3, **dadurch gekennzeichnet, daß** die Mengen der Komponenten unabhängig voneinander wie folgt sind:

| Komponente | Gew.-% |
|---|---|
| $SiO_2$ | 54,0 bis 65,0 |
| $Al_2O_3$ | 8,0 bis 21,0 |
| $P_2O_5$ | 0,5 bis 3,5 |
| $K_2O$ | 5,0 bis 8,0 |
| $Na_2O$ | 6,0 bis 11,0 |
| CaO | 2,0 bis 6,0 |
| F | 0,3 bis 1,5 |
| $B_2O_3$ | 0,2 bis 3,0 |
| $La_2O_3$ | 0 bis 2,0 |
| $Li_2O$ | 0 bis 2,0 |
| BaO | 0 bis 3,0 |
| MgO | 0 bis 3,0 |
| ZnO | 0 bis 3,0 |

(fortgesetzt)

| Komponente | Gew.-% |
|---|---|
| SrO | 0 bis 4,0 |
| TiO$_2$ | 0,5 bis 2,0 |
| ZrO$_2$ | 0,5 bis 3,0 |
| CeO$_2$ | 0,1 bis 1,5. |

5. Apatit-Glaskeramik nach einem der Ansprüche 1 bis 4, **dadurch gekennzeichnet, daß** die Apatitkristalle in ihrer größten Ausdehnung kleiner als 35 μm sind.

6. Apatit-Glaskeramik nach einem der Ansprüche 1 bis 5, **dadurch gekennzeichnet, daß** das Mol-Verhältnis von CaO : P$_2$O$_5$ : F    1: 0,020 bis 1,5 : 0,03 bis 4,2 beträgt.

7. Apatit-Glaskeramik nach einem der Ansprüche 1 bis 6, **dadurch gekennzeichnet, daß** sie einen linearen thermischen Ausdehnungskoeffizienten von 6,0 bis 12,0 $\times$ 10$^{-6}$K$^{-1}$, gemessen im Temperaturbereich von 100 °C bis 400 °C, hat.

8. Apatit-Glaskeramik nach einem der Ansprüche 1 bis 7, **dadurch gekennzeichnet, daß** sie einen CR-Wert von 0 bis 0,9 und insbesondere 0,1 bis 0,75 aufweist.

9. Verfahren zur Herstellung der Apatit-Glaskeramik gemäß einem der Ansprüche 1 bis 8, **dadurch gekennzeichnet, daß**

   a) ein Ausgangsglas, welches die Komponenten gemäß einem der Ansprüche 1 bis 3 enthält, bei Temperaturen von 1200 °C bis 1650 °C erschmolzen wird,

   b) die erhaltene Glasschmelze unter Bildung eines Glasgranulates in Wasser eingegossen wird,

   c) das Glasgranulat gegebenenfalls zu einem Glaspulver mit einer mittleren Korngröße von 1 bis 450 μm, bezogen auf die Teilchenzahl, zerkleinert wird, und

   d) das Glasgranulat oder das Glaspulver einer thermischen Behandlung von mehr als 900 °C und bis zu 1200 °C für eine Dauer von 30 Minuten bis 6 Stunden unterzogen wird.

10. Dentalmaterial, **dadurch gekennzeichnet, daß** es die Apatit-Glaskeramik gemäß einem der Ansprüche 1 bis 8 enthält.

11. Dentalmaterial nach Anspruch 10, **dadurch gekennzeichnet, daß** es zusätzlich mindestens ein Glas und/oder eine Glaskeramik der Systeme Alkali-Silicat, Alkali-Erdalkali-Silicat, Alkali-Alumosilicat, Alkali-Zink-Borosilicat, Phosphosilicat oder Alumo-Pluor-Borosilicat enthält.

12. Dentalmaterial nach Anspruch 10 oder 11, **dadurch gekennzeichnet, daß** es einen linearen thermischen Ausdehnungskoeffizienten von 5,5 bis 12,5 x 10$^{-6}$K$^{-1}$, gemessen im Temperaturbereich von 100 °C bis 400 °C, hat.

13. Verwendung des Dentalmaterials gemäß einem der Ansprüche 10 bis 12 zur Beschichtung eines Substrates und insbesondere zur Beschichtung einer dentalen Restauration.

14. Verwendung nach Anspruch 13, wobei ein Substrat auf Basis von keramischem oder glaskeramischem Werkstoff, insbesondere Lithiumdisilicat-Glaskeramik, eingesetzt wird.

15. Verwendung nach Anspruch 14, wobei die Lithiumdisilicat-Glaskeramik die folgenden Komponenten enthält:

| Komponente | Gew.-% |
|---|---|
| SiO$_2$ | 57,0 bis 80,0 |
| Al$_2$O$_3$ | 0 bis 5,0 |

(fortgesetzt)

| Komponente | Gew.-% |
|---|---|
| $La_2O_3$ | 0,1 bis 6,0 |
| MgO | 0 bis 5,0 |
| ZnO | 0 bis 8,0 |
| $K_2O$ | 0 bis 13,5 |
| $Li_2O$ | 11,0 bis 19,0 |
| $P_2O_5$ | 0 bis 11,0 |

wobei

(a) $Al_2O_3 + La_2O_3$    0,1 bis 7,0 Gew.-% und
(b) MgO + ZnO    0,1 bis 9,0 Gew.-%

ausmachen.

16. Verwendung nach einem der Ansprüche 13 bis 15, wobei das Dentalmaterial auf das Substrat aufgebracht und bei Temperaturen von 650 °C bis 1050 °C gesintert wird.

17. Geformtes Dentalprodukt, **dadurch gekennzeichnet, daß** es die Apatit-Glaskeramik gemäß einem der Ansprüche 1 bis 8 oder das Dentalmaterial gemäß einem der Ansprüche 10 bis 12 enthält.

18. Geformtes Dentalprodukt nach Anspruch 17, **dadurch gekennzeichnet, daß** es eine dentale Restauration ist.

19. Geformtes Dentalprodukt nach Anspruch 17 oder 18, **dadurch gekennzeichnet, daß** es einen Kern auf Basis von keramischem oder glaskeramischem Werkstoff und eine darauf aufgebrachte Beschichtung aus der Apatit-Glaskeramik oder dem Dentalmaterial aufweist.

20. Geformtes Dentalprodukt nach Anspruch 19, **dadurch gekennzeichnet, daß** der glaskeramische Werkstoff eine Lithiumdisilicat-Glaskeramik ist.

**Claims**

1. Translucent apatite glass ceramic, **characterised in that** it comprises the following components:

| Component | wt% |
|---|---|
| $SiO_2$ | 45.0 to 70.0 |
| $Al_2O_3$ | 5.0 to 22.0 |
| $P_2O_5$ | 0.5 to 6.5 |
| $K_2O$ | 3.0 to 8.5 |
| $Na_2O$ | 4.0 to 13.0 |
| CaO | 1.5 to 11.0 |
| F | 0.1 to 2.5 |

and the main crystalline phase is formed from needle-shaped apatite crystals.

2. Apatite glass ceramic according to Claim 1, **characterised in that** it comprises at least one of the following components:

| Component | wt% |
|---|---|
| $B_2O_3$ | 0 to 8.0 |
| $La_2O_3$ | 0 to 5.0 |

(continued)

| Component | wt% |
|-----------|-----|
| $Li_2O$ | 0 to 5.0 |
| BaO | 0 to 5.0 |
| MgO | 0 to 5.0 |
| ZnO | 0 to 5.0 |
| SrO | 0 to 7.0 |
| $TiO_2$ | 0 to 4.0 |
| $ZrO_2$ | 0 to 4.0 |
| $CeO_2$ | 0 to 3.0 |

3. Apatite glass ceramic according to Claim 1 or 2, **characterised in that** the quantities of components are, unless otherwise stated, independent of one another and as follows:

| Component | wt.% |
|-----------|------|
| $SiO_2$ | 50.0 to 68.0 |
| $Al_2O_3$ | 7.0 to 21.0 |
| $P_2O_5$ | 0.5 to 4.0 |
| $K_2O$ | 4.0 to 8.0 |
| $Na_2O$ | 4.0 to 11.0 |
| CaO | 2.0 to 8.0 |
| F | 0.2 to 2.0 |
| $B_2O_3$ | 0.2 to 4.0 |
| $La_2O_3$ | 0 to 3.0 |
| $Li_2O$ | 0 to 3.0 |
| BaO | 0 to 4.0 |
| MgO | 0 to 4.0 |
| ZnO | 0 to 4.0 |
| SrO | 0 to 5.0 |
| $TiO_2+ZrO_2$ | 0.2 to 5.0 |
| $CeO_2$ | 0 to 2.0 |

4. Apatite glass ceramic according to one of Claims 1 to 3, **characterised in that** the quantities of components are, unless otherwise stated, independent of one another and as follows:

| Component | wt.% |
|-----------|------|
| $SiO_2$ | 54.0 to 65.0 |
| $Al_2O_3$ | 8.0 to 21.0 |
| $P_2O_5$ | 0.5 to 3.5 |
| $K_2O$ | 5.0 to 8.0 |
| $Na_2O$ | 6.0 to 11.0 |
| CaO | 2.0 to 6.0 |
| F | 0.3 to 1.5 |
| $B_2O_3$ | 0.2 to 3.0 |
| $La_2O_3$ | 0 to 2.0 |
| $Li_2O$ | 0 to 2.0 |
| BaO | 0 to 3.0 |
| MgO | 0 to 3.0 |
| ZnO | 0 to 3.0 |
| SrO | 0 to 4.0 |
| $TiO_2$ | 0.5 to 2.0 |

(continued)

| Component | wt.% |
| --- | --- |
| $ZrO_2$ | 0.5 to 3.0 |
| $CeO_2$ | 0.1 to 1.5 |

5. Apatite glass ceramic according to one of Claims 1 to 4, wherein the apatite crystals are smaller than 35μm in their greatest dimension.

6. Apatite glass ceramic according to one of Claims 1 to 5, wherein the molar ratio
$CaO : P_2O_5 : F$ is       1 : 0.020 to 1.5 : 0.03 to 4.2

7. Apatite glass ceramic according to one of Claims 1 to 6, **characterised in that** it has a linear thermal expansion coefficient of 6.0 to 12.0 x $10^{-6}K^{-1}$, measured in the temperature range 100°C to 400°C.

8. Apatite glass ceramic according to one of Claims 1 to 7, **characterised in that** it has a CR value of 0 to 0.9 and, in particular, 0.1 to 0.75.

9. A process for the preparation of the apatite glass ceramic according to one of Claims 1 to 8, **characterised in that**

a) a starting glass comprising the necessary components is melted at temperatures between 1200°C and 1650°C,

b) the resulting glass melt is poured into water so as to form a glass granulate,

c) the glass granulate is optionally comminuted to a glass powder with an average particle size of 1 to 450μm, based on the number of particles, and

d) the glass granulate or the glass powder is subjected to a heat treatment at temperatures of more than 900°C and up to 1200°C for a period of 30 minutes to 6 hours.

10. Dental material, **characterised in that** it comprises the apatite glass ceramic according to one of Claims 1 to 8.

11. Dental material according to Claim 10, **characterised in that** it additionally comprises at least one glass and/or glass ceramic of the systems alkali-silicate, alkali-earth alkali-silicate, alkali-aluminosilicate, alkali-zinc-borosilicate, phosphosilicate or alumino-fluoro-borosilicate.

12. Dental material according to Claim 10 or 11, **characterised in that** it has a linear thermal expansion coefficient of 5.5 to 12.5 x $10^{-6}K^{-1}$, measured in the temperature range 100°C to 400°C.

13. Use of the dental material according to one of Claims 10 to 12 for the coating of a substrate and in particular for coating a dental restoration.

14. Use according to claim 13, wherein a substrate based on ceramic or glass ceramic material, particularly lithium disilicate glass ceramic, is used.

15. Use according to claim 14, wherein the lithium disilicate glass ceramic comprises the following components:

| Component | wt.% |
| --- | --- |
| $SiO_2$ | 57.0 to 80.0 |
| $Al_2O_3$ | 0 to 5.0 |
| $La_2O_3$ | 0.1 to 6.0 |
| MgO | 0 to 5.0 |
| ZnO | 0 to 8.0 |
| $K_2O$ | 0 to 13.5 |
| $Li_2O$ | 11.0 to 19.0 |

(continued)

| Component | wt.% |
|---|---|
| $P_2O_5$ | 0 to 11.0 |

with the proviso that

    (a) $Al_2O_3$ + $La_2O_3$ is     0.1 to 7.0 wt.% and
    (b) MgO + ZnO is     0.1 to 9.0 wt.%.

16. Use according to one of Claims 13 to 15, wherein the dental material is applied to the substrate and sintered at temperatures between 650°C and 1050°C.

17. Shaped dental product, **characterised in that** it comprises the apatite glass ceramic according to one of Claims 1 to 8 or the dental material according to one of Claims 10 to 12.

18. Shaped dental product according to Claim 17 **characterised in that** it is a dental restoration.

19. Shaped dental product according to Claim 17 or 18 **characterised in that** it has a core based on ceramic or glass ceramic material and a coating applied thereto composed of the apatite glass ceramic or the dental material.

20. Shaped dental product according to Claim 19 **characterised in that** the glass ceramic material is a lithium disilicate glass ceramic.

**Revendications**

1. Vitrocéramique à base d'apatite translucide **caractérisée en ce qu'**elle contient les composants suivants :

| Composant | % en poids |
|---|---|
| $SiO_2$ | 45,0 à 70,0 |
| $Al_2O_3$ | 5,0 à 22,0 |
| $P_2O_5$ | 0,5 à 6,5 |
| $K_2O$ | 3,0 à 8,5 |
| $Na_2O$ | 4,0 à 13,0 |
| CaO | 1,5 à 11,0 |
| F | 0,1 à 2,5 |

et la phase cristalline principale est constituée de cristaux d'apatite aciculaires.

2. Vitrocéramique à base d'apatite selon la revendication 1, **caractérisée en ce qu'**elle contient au moins un des composants suivants :

| Composant | % en poids |
|---|---|
| $B_2O_3$ | 0 à 8,0 |
| $La_2O_3$ | 0 à 5,0 |
| $Li_2O$ | 0 à 5,0 |
| BaO | 0 à 5,0 |
| MgO | 0 à 5,0 |
| ZnO | 0 à 5,0 |
| SrO | 0 à 7,0 |
| $TiO_2$ | 0 à 4,0 |
| $ZrO_2$ | 0 à 4,0 |
| $CeO_2$ | 0 à 3,0 |

**3.** Vitrocéramique à base d'apatite selon la revendication 1 ou 2, **caractérisée en ce que** les quantités des composants, à moins d'indication contraire, indépendamment les unes des autres, sont comme suit :

| Composant | % en poids |
|---|---|
| $SiO_2$ | 50,0 à 68,0 |
| $Al_2O_3$ | 7,0 à 21,0 |
| $P_2O_5$ | 0,5 à 4,0 |
| $K_2O$ | 4,0 à 8,0 |
| $Na_2O$ | 4,0 à 11,0 |
| CaO | 2,0 à 8,0 |
| F | 0,2 à 2,0 |
| $B_2O_3$ | 0,2 à 4,0 |
| $La_2O_3$ | 0 à 3,0 |
| $Li_2O$ | 0 à 3,0 |
| BaO | 0 à 4,0 |
| MgO | 0 à 4,0 |
| ZnO | 0 à 4,0 |
| SrO | 0 à 5,0 |
| $TiO_2 + ZrO_2$ | 0,2 à 5,0 |
| $CeO_2$ | 0 à 2,0 |

**4.** Vitrocéramique à base d'apatite selon l'une quelconque des revendications 1 à 3, **caractérisée en ce que** les quantités des composants, indépendamment les unes des autres, sont comme suit :

| Composant | % en poids |
|---|---|
| $SiO_2$ | 54,0 à 65,0 |
| $Al_2O_3$ | 8,0 à 21,0 |
| $P_2O_5$ | 0,5 à 3,5 |
| $K_2O$ | 5,0 à 8,0 |
| $Na_2O$ | 6,0 à 11,0 |
| CaO | 2,0 à 6,0 |
| F | 0,3 à 1,5 |
| $B_2O_3$ | 0,2 à 3,0 |
| $La_2O_3$ | 0 à 2,0 |
| $Li_2O$ | 0 à 2,0 |
| BaO | 0 à 3,0 |
| MgO | 0 à 3,0 |
| ZnO | 0 à 3,0 |
| SrO | 0 à 4,0 |
| $TiO_2$ | 0,5 à 2,0 |
| $ZrO_2$ | 0,5 à 3,0 |
| $CeO_2$ | 0,1 à 1,5 |

**5.** Vitrocéramique à base d'apatite selon l'une quelconque des revendications 1 à 4, **caractérisée en ce que** les cristaux d'apatite ont dans leur plus grande dimension une taille inférieure à 35 µm.

**6.** Vitrocéramique à base d'apatite selon l'une quelconque des revendications 1 à 5, **caractérisée en ce que** le rapport molaire
$CaO:P_2O_5:F$ est de 1:0,020 à 1,5: 0,03 à 4,2.

**7.** Vitrocéramique à base d'apatite selon l'une quelconque des revendications 1 à 6, **caractérisée en ce qu'**elle a un coefficient de dilatation thermique linéaire de 6,0 à 12,0 x $10^{-6}K^{-1}$, mesuré dans la plage de température allant de 100°C à 400°C.

**8.** Vitrocéramique à base d'apatite selon l'une quelconque des revendications 1 à 7, **caractérisé en ce qu'**elle présente un indice CR de 0 à 0,9 et en particulier de 0,1 à 0,75.

**9.** Procédé pour la fabrication de la vitrocéramique à base d'apatite selon l'une quelconque des revendications 1 à 8, **caractérisé en ce que**

a) on fait fondre à des températures de 1 200 à 1 650°C un verre de départ qui contient les composants selon l'une quelconque des revendication 1 à 3,
b) on verse dans de l'eau le verre fondu obtenu, avec formation d'un verre granulé,
c) on fragmente éventuellement le verre granulé en une poudre de verre ayant une taille moyenne de particules de 1 à 450 μm, par rapport au nombre des particules, et
d) on soumet le verre granulé ou la poudre de verre à un traitement thermique de plus de 900°C à 1 200°C, pendant une durée de 30 minutes à 6 heures.

**10.** Matériau dentaire **caractérisé en ce qu'**il contient la vitrocéramique à base d'apatite selon l'une quelconque des revendications 1 à 8.

**11.** Matériau dentaire selon la revendication 10, **caractérisé en ce qu'**il contient en outre au moins un verre et/ou une vitrocéramique des systèmes silicate-métal alcalin, silicate-métal alcalin-métal alcalino terreux, aluminosilicate-métal alcalin, borosilicate de zinc-métal alcalin, phosphosilicate ou alumino-fluoro-borosilicate.

**12.** Matériau dentaire selon la revendication 10 ou 11, **caractérisé en ce qu'**il a un coefficient de dilation thermique linéaire de 5,5 à 12,5 x $10^{-6}K^{-1}$, mesuré dans la plage de température de 100°C à 400°C.

**13.** Utilisation du matériau dentaire selon l'une quelconque des revendications 10 à 12, pour les revêtements d'un support et en particulier pour le revêtement d'une restauration dentaire.

**14.** Utilisation selon la revendication 13, dans laquelle on utilise un support à base d'un matériau céramique ou vitrocéramique, en particulier une vitrocéramique au disilicate de lithium.

**15.** Utilisation selon la revendication 14, dans laquelle la vitrocéramique au disilicate de lithium contient les composants suivants :

| Composant | % en poids |
|---|---|
| $SiO_2$ | 57,0 à 80,0 |
| $Al_2O_3$ | 0 à 5,0 |
| $La_2O_3$ | 0,1 à 6,0 |
| MgO | 0 à 5,0 |
| ZnO | 0 à 8,0 |
| $K_2O$ | 0 à 13,5 |
| $Li_2O$ | 11,0 à 19,0 |
| $P_2O_5$ | 0 à 11,0 |

où

(a) $Al_2O_3$ + $La_2O_3$ représentent     0,1 à 7,0 % en poids et
(b) MgO + ZnO représentent     0,1 à 9,0 % en poids

**16.** Utilisation selon l'une quelconque des revendications 13 à 15, dans laquelle le matériau dentaire est appliqué sur le support et fritté à des températures de 650°C à 1 050°C.

**17.** Produit dentaire façonné, **caractérisé en ce qu'**il contient la vitrocéramique à base d'apatite selon l'une quelconque des revendications 1 à 8 ou le matériau dentaire selon l'une quelconque des revendications 10 à 12.

**18.** Produit dentaire façonné selon la revendication 17, **caractérisé en ce qu'**il s'agit d'une restauration dentaire.

19. Produit dentaire façonné selon la revendication 17 ou 18, **caractérisé en ce qu'**il présente un noyau à base de céramique ou de matériau vitrocéramique et un revêtement appliqué sur celui-ci, à base de la vitrocéramique à apatite ou du matériau dentaire.

20. Produit dentaire façonné selon la revendication 19, **caractérisé en ce que** le matériau vitrocéramique est une vitrocéramique au disilicate de lithium.